# EUROPÄISCHE PATENTSCHRIFT

(11) **EP 2 359 891 B1**
(45) Veröffentlichungstag und Bekanntmachung des Hinweises auf die Patenterteilung: **23.01.2013**
(21) Anmeldenummer: 10450019.4
(22) Anmeldetag: 16.02.2010
(51) Int. Cl.: A61M 25/10

(54) **Steuer- und Aufblasgerät für einen Ballonkatheter**
Control and inflation device for a balloon catheter
Appareil de guidage et de gonflage pour un cathéter à ballonnet

(43) Veröffentlichungstag der Anmeldung: 24.08.2011
(73) Patentinhaber: Miracor Medical Systems GmbH, 1090 Wien (AT)
(72) Erfinder: Hoem, Jon H., 6315 Oberaegeri (CH); Kohr, Oliver, 3076 Worb (CH)
(74) Vertreter: Haffner und Keschmann Patentanwälte KG

(56) Entgegenhaltungen:
- US-A- 3 275 001
- US-A- 3 675 658
- US-A- 5 158 529
- US-A- 5 486 192
- US-B1- 6 179 815

## Beschreibung

Die Erfindung betrifft ein Steuer- und Aufblasgerät für einen Ballonkatheter umfassend einen Anschluss für ein Aufblaslumen eines Ballonkatheters, einen Druckbehälter, einen Vakuumtank und eine Pumpe wobei der Druckbehälter über eine mit einem Schaltventil versehene Leitung mit dem Anschluss für das Aufblaslumen verbunden ist und der Vakuumtank über eine mit einem Schaltventil versehene Leitung mit dem Anschluss für das Aufblaslumen verbunden ist sodass der Anschluss für das Aufblaslumen abwechselnd mit dem Vakuumtank und mit dem Druckbehälter verbindbar ist.

Ein derartiges Gerät ist der US 5,486,192 zu entnehmen.

Bei einer Reihe von medizinischen Anwendungen kommen Ballonkatheter zum Einsatz. Ballonkatheter weisen einen Ballon auf, der durch Einbringen eines Fluids vom kollabierten Zustand in den expandierten Zustand und durch Entleeren vom expandierten Zustand wieder zurück in den kollabierten Zustand gebracht werden kann. Als Fluid kommen Gase oder Flüssigkeiten in Frage.

Ballonkatheter werden beispielsweise für die Ballondilatation verengter Blutgefäße im Rahmen einer perkutanen transluminalen Angioplastie eingesetzt. Dabei wird ein an einem Gefäßkatheter angebrachter Ballon innerhalb eines Blutgefäßes bis zu einer krankhaft verengten Gefäßstelle vorgeschoben und der Ballon an der verengten Stelle unter hohem Druck (6 bis 20 bar) entfaltet. Dadurch werden die Engstellen, die vor allem durch arteriosklerotische Gefäßverkalkung entstehen, so gedehnt, dass sie den Blutstrom nicht mehr oder weniger stark behindern.

Ballonkatheter können aber auch im Rahmen einer druckgesteuerten intermittierenden Okklusion eines Körpergefäßes, insbesondere des Koronarsinus, zum Einsatz gelangen. Verfahren zur druckgesteuerten intermittierenden Okklusion des Koronarsinus sind beispielsweise in den Dokumenten EP 609914 A2, EP 230996 A2, EP 1406683 A2, EP 1753483 A1, EP 1755702 A1 und WO 2008/064337 A1 beschrieben. Bei diesen Verfahren wird der Koronarsinus mit Hilfe eines Ballons zyklisch okkludiert und wieder freigegeben, wobei das Okkludieren des Koronarsinus während der Okklusionsphasen einen Druckanstieg und in der Folge eine Retroperfusion von Blut über die entsprechende Vene in die nutritiven Kapillaren des Ischämiegebiets bewirkt, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden. Während der Okklusionsphasen wird jeweils der Druck im okkludierten Koronarsinus gemessen, und das Aufheben der Okklusion ebenso wie das Einleiten der Okklusion erfolgt in Abhängigkeit der Druckmesswerte.

Im Gegensatz zur Ballondilatation im Rahmen einer perkutanen transluminalen Angioplastie ist es bei der druckgesteuerten intermittierenden Okklusion eines Blutgefäßes, und insbesondere des Koronarsinus, nicht das Ziel, den Ballon mit einem derartig hohen Druck zu beaufschlagen, dass eine irreversible Verformung bzw. Beschädigung, und insbesondere Ausdehnung des entsprechenden Gefäßbereichs erfolgt. Vielmehr soll das Aufblasen des Ballons in einer Weise gesteuert werden, dass der Ballon auf die Gefäßwand einen Druck ausübt, der gerade dazu ausreicht, das Blutgefäß mit einer hinreichenden Sicherheit zu verschließen und ein Vorbeifließen von Blut am Ballon zu verhindern. Wenn der Ballon mit einem zu hohen Druck beaufschlagt wird, hat dies eine zu starke radiale Ausdehnung im Blutgefäß zur Folge, wobei die entsprechende mechanische Belastung der Gefäßwand zu irreversiblen Schäden führen kann, was vermieden werden soll. Andererseits würde eine zu geringe Druckbeaufschlagung des Ballons dazu führen, dass zwar die Gefäßwand geschont wird, der Ballon das Gefäß aber nicht vollständig verschließt.

Die Erfindung zielt daher darauf ab, das Befüllen des Ballons so zu steuern, dass die Gefäßwand nicht überbeansprucht wird, gleichzeitig jedoch ein sicheres Verschließen des Blutgefäßes erreicht wird. Dabei muss vor allem auf die patienten- und/oder katheterabhängig unterschiedlichen Gegebenheiten Rücksicht genommen werden, wobei dies insbesondere den unterschiedlichen Gefäßdurchmesser, den unterschiedlichen Blutdruck, der sich im okkludierten Gefäß aufbaut, und die unterschiedliche Gefäßviskoelastizität der einzelnen Patienten bzw. die z.B. produktionsbedingt unterschiedlichen Kathetervolumina betrifft. Insbesondere soll erfindungsgemäß ein Steuer- und Aufblasgerät für einen Ballonkatheter geschaffen werden, mit welchem den obigen Vorgaben entsprochen werden kann. Dabei soll die für medizinische Geräte erforderliche Betriebssicherheit gewährleistet sein, wobei insbesondere verhindert werden soll, dass bei einer allfälligen Fehlfunktion das für das Aufblasen des Ballonkatheters verwendete Fluid in das entsprechende Körpergefäß gelangt.

Es wird ein Verfahren zum Bestimmen einer patienten- und/oder katheterspezifisch optimierten Fluidmenge zum Befüllen eines Ballons eines Ballonkatheters zum Aufblasen desselben angegeben, umfassend die Schritte
a) Evakuieren des Ballons,
b) Vorsehen einer definierten Startmenge an Fluid und Verwenden dieser Startmenge zum Befüllen des Ballons sowie Messen des Start-Ballondrucks,
c) Vorsehen einer im Vergleich zur vorhergehenden Menge erhöhten Menge an Fluid und Verwenden der erhöhten Menge zum Befüllen des Ballons sowie Messen des durch die Befüllung erzielten Ballondrucks oder von Schwankungen des Ballondrucks,
d) Vergleichen des gemessenen Ballondrucks oder der Schwankungen des Ballondrucks mit einem vorgegebenen Sollwert und Wiederholen des Schrittes c) bis der Ballondruck oder die Schwankungen den Sollwert erreicht haben,
e) Speichern der zuletzt vorgesehenen Menge an Fluid als Referenzwert für das Aufblasen des Ballons.

Das Verfahren dient dem Bestimmen einer patienten- und/oder katheterspezifisch optimierten Fluidmenge zum Befüllen des Ballons im Zuge der Vorbereitung des eigentlichen Okklusionsvorgangs. Vor der Durchführung der eigentlichen Behandlung des Patienten wird mit dem Verfahren die optimale Fluidmenge zum Befüllen des Ballons festgestellt, wobei die ermittelte optimale Fluidmenge als Referenzwert für die nachfolgende Behandlung des Patienten verwendet wird und auf diese Art und Weise das Steuer- und Aufblasgerät für den Ballonkatheter auf einen patienten- bzw. katheterspezifischen Wert eingestellt wird. Während der nachfolgenden Okklusionszyklen wird der mit Hilfe des Verfahrens ermittelte Wert beibehalten oder an während der Behandlung auftretende geänderte Verhältnisse angepasst. Eine Nachstellung des patienten- und/oder katheterspezifisch ermittelten Werts kann beispielsweise dann indiziert sein, wenn sich die Druckverhältnisse im okkludierten Gefäß ändern, wenn sich die Viskoelastizität des Gefäßes ändert oder wenn die Behandlung an eine andere Stelle des Gefäßes verlegt wird.

Am Anfang des Verfahrens wird der Ballon evakuiert, um den Ballon auf ein definiertes Druckniveau zu bringen, das Ausgangspunkt für die nachfolgenden Verfahrensschritte ist. Gemäß Schritt b) wird eine definierte Startmenge an Fluid vorgesehen und es wird diese Startmenge zum Befüllen des Ballons verwendet, worauf der sich aufgrund der Startmenge ergebende Ballondruck gemessen wird. Gemäß Schritt c) wird die vorhergehende Fluidmenge nun erhöht und es wird der sich aufgrund der erhöhten Fluidmenge ergebende Ballondruck gemessen. Der ermittelte Ballondruck wird dabei mit einem vorgegebenen Sollwert verglichen und es wird die für das Befüllen des Ballons verwendete Fluidmenge stufenweise erhöht, bis der Ballondruck den vorgegebenen Sollwert erreicht hat. Als Sollwert kann dabei patientenunabhängig beispielsweise ein absoluter Wert von mindestens 70 mm Hg, insbesondere ungefähr 80 mm Hg gewählt werden, wobei der Sollwert ein Okkludieren des Gefäßes mit dem Ballon sicherstellen soll. Der Sollwert kann aber auch patientenabhängig gewählt werden und beispielsweise 10 % über dem sich beim jeweiligen Patienten während der Okklusion ergebenden Maximaldruck im verschlossenen Gefäß liegen.

Zur Erreichung des vorgegebenen Sollwerts ist je nach der Gefäßviskoelastizität und anderen patientenabhängigen Faktoren bzw. je nach Katheter ein unterschiedlich starkes Aufblasen des Ballons, d.h. eine unterschiedlich große Menge an eingebrachtem Fluid erforderlich. Dadurch, dass ein stufenweises Herantasten beim Einbringen des Fluids in den Ballon vorgenommen wird, wird verhindert, dass der Druck im Ballon den vorgegebenen Sollwert überschreitet. Dadurch, dass die dem Sollwert entsprechende Fluidmenge, die in den Ballon eingebracht wird, als Referenzwert festgelegt wird, ist es beim nachfolgenden Aufblasvorgang entbehrlich, eine Druckmessung im Ballon durchzuführen, da der Aufblasvorgang ausschließlich aufgrund der eingebrachten Fluidmenge gesteuert wird. Durch das Begrenzen der eingebrachten Fluidmenge kann eine einfache Regelung des Aufblasvorgangs erreicht werden und es ist insbesondere sichergestellt, dass unzulässige Betriebszustände, wie insbesondere unzulässige Druckzustände auftreten. Wenn während nachfolgender Aufblasvorgänge der Referenzwert nachjustiert werden soll, muss der Druck im Ballon laufend ermittelt und Abweichungen vom Sollwert erfasst werden, wobei der Referenzwert angepasst wird, wenn die erfasste Abweichung einen vorgegebenen Toleranzwert überschreitet.

Eine laufende Druckmessung ist auch nützlich, wenn der Ballondruck nicht nur hinsichtlich der Einhaltung des Sollwerts, sondern auch hinsichtlich des Überschreitens eines sicherheitsrelevanten oberen Grenzwerts überwacht werden soll. Ein derartiger oberer Grenzwert orientiert sich dabei an Werten, wie sie für die Betriebssicherheit des Katheters z.B. hinsichtlich eines Platzens als zulässig befunden werden, und kann beispielsweise zwischen 90 und 120 mm Hg betragen. Bei Überschreiten des oberen Grenzwerts muss der Betrieb des Systems abgebrochen werden.

Gemäß einer alternativen Vorgehensweise wird nicht der absolute Wert des Ballondrucks gemessen und mit einem Sollwert verglichen, sondern es werden Schwankungen des Ballondrucks erfasst und ausgewertet. Druckschwankungen können nämlich beispielsweise dadurch auftreten, dass der Ballon soweit aufgeblasen wird, dass er die Gefäßwand des Patienten berührt und dabei bei der Annäherung an die Gefäßwand aufgrund des Blutflusses im schmalen Spalt zwischen dem Ballon und der Gefäßwand Strömungen entstehen, die Druckschwankungen im Ballon bewirken. Solche Druckschwankungen sind somit charakteristisch dafür, dass der Ballon die Gefäßwand erreicht.

Gemäß einer bevorzugten Weiterbildung ist vorgesehen, dass die Schritte b) - d) das Erstellen einer Druck-Volumen-Kurve aus dem sich aus der Verwendung der Startmenge ergebenden Start-Ballonvolumen und dem Start-Ballondruck sowie der sich jeweils aus der Verwendung der stufenweise erhöhten Fluidmengen ergebenden Ballonvolumina und der jeweiligen Ballondrücke umfassen. Anhand der Druck-Volumen-Kurve lässt sich in einfacher Art und Weise das stufenweise Herantasten an die optimale Befüllung des Ballons beobachten und nachvollziehen, wobei der Verlauf der Druck-Volumen-Kurve in der Regel derart ausgestaltet ist, dass in einem ersten Bereich, in welchem der Ballon die Gefäßwand noch nicht berührt, das eingebrachte Fluidvolumen bei nahezu gleich bleibendem oder leicht steigendem Druck steigt und in einem zweiten Bereich, bei welchem der Ballon sich an die Gefäßwand anlegt, der Balloninnendruck bei jeder Erhöhung des eingebrachten Fluidvolumens signifikant ansteigt.

Gemäß einer besonders bevorzugten Verfahrensweise wird derart vorgegangen, dass der Ballon vor jeder Durchführung oder Wiederholung des Schritts c) evakuiert wird. Dadurch wird zunächst sichergestellt, dass für jede der im Zuge des Herantastens an den Ballondrucksollwert stufenweisen Erhöhungen der in den Ballon eingebrachten Fluidmenge ein definierter Ausgangszustand vorliegt, wobei der Ballon nach jedem Evakuieren mit einer im Vergleich zur vorhergehenden Menge erhöhten Fluidmenge befüllt wird. Um bei jedem Evakuierungsvorgang den gleichen Evakuierungszustand zu erreichen, wird dabei bevorzugt derart vorgegangen, dass der Ballondruck während des Evakuieren des Ballons gemessen wird und das Evakuieren so lange vorgenommen wird, bis ein vorgegebener Unterdruck erreicht ist, wobei der vorgegebene Unterdruck bei jedem Evakuieren vorzugsweise gleich gewählt wird. Zusätlich kann der Druck während des Evakuierens auch in der für das Evakuieren verantwortlichen Unterdruckquelle, vorzugsweise einem Vakuumtank, gemessen werden, um das Erreichen des vorgegebenen Unterdrucks zu überwachen.

Das Evakuieren des Ballons vor jeder Erhöhung der in den Ballon eingebrachten Fluidmenge führt auch dazu, dass der Ballon im Zuge des Verfahrens regelmäßig und zuverlässig kollabiert, wodurch verhindert wird, dass der Fluss in dem betroffenen Körpergefäß zu lange behindert oder unterbrochen wird.

Gemäß einer weiteren bevorzugten Vorgehensweise ist vorgesehen, dass die Erhöhung der Fluidmenge stufenweise um einen vorzugsweise bei jeder Erhöhung gleichen Wert vorgenommen wird.

Das Aufblasen des Ballons erfolgt gemäß einer bevorzugten Vorgehensweise dadurch, dass die Startmenge an Fluid und die jeweils erhöhte Fluidmenge in einen Druckbehälter dosiert wird und der Ballon lediglich auf Grund eines vollständigen Druckausgleichs zwischen dem Druckbehälter und dem Ballon gefüllt wird. Das Fluid wird somit nicht direkt in den Ballon gefüllt, da dies die Gefahr bergen würde, dass eine unkontrollierte Menge an Fluid aufgrund einer Fehlfunktion in den Ballon gelangt. Wenn nun, wie dies bevorzugt vorgesehen ist, das Fluid zuerst in den Druckbehälter dosiert wird, kann die Fluidmenge genau kontrolliert werden und eine Fehlfunktion würde lediglich dazu führen, dass der Druckbehälter mit einer überhöhten Menge befüllt wird, was aber vor dem Aufblasen des Ballons festgestellt bzw. kontrolliert werden kann. Wenn nun die jeweils vorgesehene Menge an Fluid in den Druckbehälter dosiert werde, erfolgt das Befüllen des Ballons lediglich dadurch, dass die Verbindung zwischen dem Druckbehälter und dem Ballon geöffnet wird und sich der Ballon daher lediglich aufgrund eines vollständigen Druckausgleichs zwischen dem Druckbehälter und dem Ballon füllt.

Dabei kann bevorzugt so vorgegangen werden, dass der im Druckbehälter gemessene Druck zur Regelung der Dosierung der jeweils gewünschten Fluidmenge in den Druckbehälter verwendet wird. Bezüglich der als Referenzmenge gespeicherten Fluidmenge bedeutet dies, dass der sich aus der Befüllung des Druckbehälters mit der Referenzmenge ergebende Druck als Referenzdruck gespeichert wird und die Befüllung bis zum Erreichen des Referenzdrucks vorgenommen wird, sodass die Regelung des Befüllvorganges des Druckbehälters vereinfacht wird.

Gemäß einer weiteren bevorzugten Vorgehensweise ist vorgesehen, dass der Ballondruck über ein von dem für die Befüllung vorgesehenen Katheterlumen gesondertes Katheterlumen gemessen wird. Dies führt zu einer erhöhten Sicherheit insbesondere dann, wenn der Ballondruck auch in dem für die Befüllung vorgesehenen Katheterlumen gemessen wird, Auf diese Art und Weise stehen zwei unabhängige Druckmesswerte zur Verfügung und es kann aus einem Vergleich dieser Druckmesswerte auf allfällige Fehlfunktionen geschlossen werden.

Weiters wird ein Verfahren zum Aufblasen und Entleeren eines Ballons eines Ballonkatheters angegeben, bei welchem der Ballon zur Aufblasen mit einer Überdruckquelle und zum Entleeren mit einer Unterdruckquelle verbunden wird, und welches sich dadurch auszeichnet, dass als Überdruckquelle ein Druckbehälter vorgesehen ist, der mit Fluid unter Überdruck befüllt wird, und dass das Aufblasen des Ballons ausschließlich auf Grund eines vollständigen Druckausgleichs zwischen dem Druckbehälter und dem Ballon erfolgt. Dadurch, dass das Aufblasen des Ballons ausschließlich aufgrund eines vollständigen Druckausgleichs zwischen dem Druckbehälter und dem Ballon erfolgt, werden Fehlfunktionen wie insbesondere das Überfüllen des Ballons über den vorgesehenen Höchstdruck hinaus wirksam vermieden. Der Druckbehälter wird vorab mit einer vorgegebenen Fluidmenge befüllt, wobei die Fluidmenge derart bemessen wird, dass sich ein vorgegebener Befüllungszustand und Druckzustand im Ballon ergibt. Wenn nun die Verbindung zwischen dem vorgefüllten Druckbehälter und dem Ballon geöffnet wird, stellt sich aufgrund des vollständigen Druckausgleichs zwischen dem Druckbehälter und dem Ballon ein vorgegebener Befüllungszustand des Ballons ein, sodass das Körpergefäß verschlossen wird. Dadurch, dass die Befüllung des Ballons ausschließlich aufgrund eines vollständigen Druckausgleichs zwischen dem Druckbehälter und dem Ballon erfolgt, sind gesonderte Regelungs- oder Kontrollvorgänge während des Befüllens entbehrlich und es wird ein unzulässiger Befüllzustand des Ballons insbesondere dadurch verhindert, dass lediglich die vorab in den Druckbehälter dosierte Fluidmenge aufgrund des anschließenden Druckausgleichs in die Verbindungsleitung und den Ballon gelangt.

Eine zusätzliche Kontrolle kann bevorzugt dahingehend vorgenommen werden, dass die Inflationszeit überwacht wird. Wenn die Inflationszeit unter einem vorgegebenen unteren Grenzwert liegt, z.B. 0,2 sec., dann wird eine Fehlermeldung generiert bzw. das System ausgeschaltet. Ein zu schnelles Aufblasen könnte nämlich zu einer Beschädigung des Gefäßes führen. Wenn die Inflationszeit über einem vorgegebenen oberen Grenzwert liegt, z.B. 0,5 sec., dann wird ebenfalls eine Fehlermeldung generiert bzw. das System ausgeschalten. Ein zu langsames Aufblasen würde nämlich dazu führen, dass der Blutfluss im Gefäß zu lange gehindert wird.

Eine weitere Kontrolle kann bevorzugt dahingehend vorgenommen werden, dass die Deflationszeit überwacht wird. Wenn die Deflationszeit unter einem vorgegebenen unteren Grenzwert liegt, z.B. 0,5 sec., dann wird eine Fehlermeldung generiert bzw. das System ausgeschaltet. Ein zu schnelles Kollabieren des Ballons könnte nämlich zu einer Beschädigung des Gefäßes führen.

Ein Zyklus bestehend aus Entleeren und Aufblasen des Ballons wird gemäß einer bevorzugten Weiterbildung mit Vorteil durch eine Abfolge der folgenden Schritte vorgenommen:
a) Verbinden des Ballons mit einer Unterdruckquelle, um den Ballon zu evakuieren, insbesondere auf einen vorgegebenen Unterdruck,
b) Unterbrechen der Verbindung zwischen dem Ballon und der Unterdruckquelle,
c) Dosieren einer vorgegebenen Fluidmenge in den Druckbehälter, wodurch dieser mit Fluid unter Überdruck befüllt wird,
d) Öffnen der Verbindung zwischen dem Druckbehälter und dem Ballon, wobei das Aufblasen des Ballons ausschließlich auf Grund eines vollständigen Druckausgleichs zwischen dem Druckbehälter und dem Ballon erfolgt,
e) Schließen der Verbindung zwischen dem Druckbehälter und dem Ballon, nachdem ein vollständiger Druckausgleich stattgefunden hat.

Als Unterdruckquelle findet bevorzugt ein Vakuumtank Verwendung, wobei das Fluid zwischen Vakuumtank, Druckbehälter und Ballon bevorzugt im Kreislauf geführt wird, was den Vorteil mit sich bringt, dass auch bei einer Vielzahl von Aufblas- und Entleerungszyklen dem Kreislauf kein neues Fluid zugeführt werden muss. Eine vorteilhafte Verfahrensweise sieht in diesem Zusammenhang vor, dass das Befüllen des Druckbehälters das Absaugen von Fluid aus einem die Unterdruckquelle ausbildenden Vakuumtank umfasst.

Weiters ist bevorzugt vorgesehen, dass das Befüllen des Druckbehälters mit Hilfe einer zwischen dem Druckbehälter und dem Vakuumtank angeordneten Pumpe erfolgt.

Mit Vorteil wird das Verfahren derart durchgeführt, dass das den Druckbehälter, die Pumpe, den Vakuumtank und den Ballon sowie die Verbindungsleitungen umfassende Fluidsystem mit einer Menge von Fluid gefüllt wird, sodass der Druck innerhalb des Systems bei einem vollständigen Druckausgleich aller Komponenten weniger als 2 bar, bevorzugt weniger als 1 bar beträgt. Dies hat eine wesentliche Erhöhung der Betriebssicherheit zur Folge, da sichergestellt ist, dass eine unkontrollierte Fluidmenge auch dann nicht in den Ballon gelangt, wenn sämtliche Sicherheitsventile oder andere Sicherheitseinrichtungen versagen. Im äußersten Fall würde bei einem Versagen von Ventilen innerhalb des gesamten Systems ein Druckausgleich stattfinden, wobei sich aufgrund der Gesamtmenge des sich innerhalb des Systems befindlichen Fluids ein Druck von < 2 bar ergeben würde, sodass sich ein entsprechend kollabierter Zustand des Ballons ergeben würde.

Zur Lösung der oben angegebenen Aufgabe wird gemäß der Erfindung ein Steuer- und Aufblasgerät für einen Ballonkatheter angegeben, umfassend einen Anschluss für ein Aufblaslumen eines Ballonkatheters, einen Druckbehälter, einen Vakuumtank und eine Pumpe, wobei Schaltventile derart angeordnet sind, dass der Anschluss für das Aufblaslumen abwechselnd mit dem Vakuumtank und mit dem Druckbehälter verbindbar ist. Vorzugsweise ist dabei vorgesehen, dass der Druckbehälter abwechselnd mit dem Anschluss für das Aufblaslumen und über die Pumpe mit dem Vakuumtank verbindbar ist.

Bevorzugt ist die Ausbildung derart weitergebildet, dass ein Anschluss für ein vom Aufblaslumen gesondertes Lumen des Katheters vorgesehen ist, der mit einer Druckmesseinrichtung verbunden ist.

Bevorzugt bilden der Druckbehälter, die Pumpe und der Vakuumtank mit dem Aufblaslumen des Katheters einen geschlossenen Fluidkreislauf aus.

Bevorzugt weist der geschlossene Kreislauf einen Anschluss zum Befüllen des Kreislaufes mit Fluid auf.

Bevorzugt ist an den Vakuumtank eine Kondensatpumpe angeschlossen.

Außerdem wird ein Steuer- und Aufblasgerät für einen Ballonkatheter angegeben, umfassend einen Druckbehälter für Fluid, eine Dosiereinrichtung zum Dosieren von Fluid in den Druckbehälter, eine Evakuierungseinrichtung zum Evakuieren des Druckbehälters, einen mit dem Druckbehälter über ein schaltbares Ventil in Verbindung bringbaren Anschluss für ein Aufblaslumen des Ballonkatheters, wenigstens eine Druckmesseinrichtung für den im Ballon des Ballonkatheters herrschenden Fluiddruck und eine Steuerschaltung, der die Messwerte der Druckmesseinrichtung zugeführt sind und die mit der Dosiereinrichtung zum Dosieren einer definierten Fluidmenge in den Druckbehälter in Abhängigkeit von den Druckmesswerten zusammenwirkt.

Eine bevorzugte Weiterbildung sieht vor, dass die Steuerschaltung mit dem schaltbaren Ventil derart zusammenwirkt, dass das schaltbare Ventil geschlossen ist, wenn die Dosiervorrichtung Fluid in den Druckbehälter dosiert. Die Dosiervorrichtung kann beispielsweise von einer Pumpe gebildet sein.

Weiters kann bevorzugt vorgesehen sein, dass eine Druckmesseinrichtung für den im Druckbehälter herrschenden Fluiddruck vorgesehen ist, dessen Messwerte der Steuerschaltung zugeführt sind, dass ein Speicher für einen oberen Drucksollwert vorgesehen ist und dass die Steuerschaltung derart mit der Dosiervorrichtung zusammenwirkt, dass das Dosieren von Fluid in den Druckbehälter beendet wird, wenn der im Druckbehälter gemessene Druck den Drucksollwert erreicht.

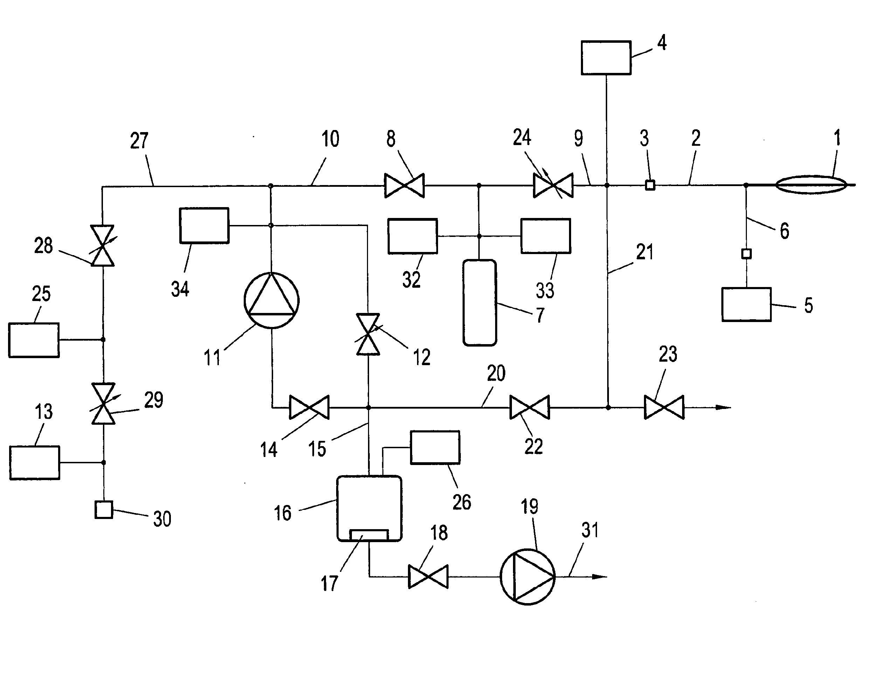

Die Erfindung wird nachfolgend anhand eines in der Zeichnung schematisch dargestellten Ausführungsbeispiels näher erläutert. In der Figur ist ein Blockschaltbild eines Steuer- und Aufblasgeräts für einen Ballonkatheter dargestellt.

Der Ballon des Ballonkatheters ist mit 1 bezeichnet. Der Ballon ist über ein Aufblaslumen 2 an den Anschluss 3 des Steuer-und Aufblasgeräts angeschlossen. Der Druck im Aufblaslumen 2 kann über eine schematisch dargestellte Druckmesseinrichtung 4 gemessen werden. Über eine weitere Druckmesseinrichtung 5 kann ein weiterer Messwert für den Ballondruck gewonnen werden. Die Druckmesseinrichtung 5 ist dabei über ein gesondertes Druckmesslumen 6 mit dem Ballon 1 verbunden.

Das Steuer- und Aufblasgerät weist einen Druckbehälter 7 auf, der über die Ventile 8 bzw. 24 entweder über die Leitung 9 mit dem Anschluss 3 oder über die Leitung 10 mit dem Fluidkreislauf verbunden werden kann. Mit 32 und 33 sind Druckmesseinrichtungen zum Messen des im Druckbehälter 7 herrschenden Drucks bezeichnet. Es ist weiters eine Pumpe 11 vorgesehen, zu welcher parallel ein Absperrventil 12 geschaltet ist. Eine leitende Verbindung zwischen der Pumpe 11 und dem Vakuumtank 16 kann über ein Sperrventil 14 und eine Leitung 15 hergestellt werden. Das sich im Vakuumtank 16 gegebenenfalls ansammelnde Kondensat ist schematisch mit 17 dargestellt und kann über ein Sperrventil 18 und mit Hilfe der Kondensatpumpe 19 abgepumpt werden. Die Verbindungsleitungen 20 und 21 können über das Absperrventil 22 in leitende Verbindung mit dem Vakuumtank 16 gebracht werden. Zur Ermittlung des Drucks im Vakuumtank 16 ist eine Druckmesseinrichtung 26 vorgesehen.

Ein Notfallventil ist mit 23 bezeichnet.

Die Einspeisung für den Fluidkreislauf erfolgt über die Leitung 27, an welche über ein Drosselventil 28 und ein Absperrventil 29 ein Fluidvorratsbehälter 30, wie beispielsweise ein Heliumzylinder, angeschlossen werden kann.

Die Funktionsweise des Steuer- und Aufblasgeräts wird nun näher erläutert.

Set-up Modus:
Im Set-up Modus sind zunächst alle Komponenten des Systems mit Luft od. dgl. gefüllt und die Ventile sind geschlossen.

Evakuierung:
Im Evakuierungsmodus wird nun das gesamte System entleert. Zu diesem Zweck sind die Absperrventile 23 und 29 geschlossen,
wohingegen alle anderen Ventile geöffnet sind. Die sich im System gegebenenfalls befindliche Luft wird mit Hilfe der Kondensatpumpe 19 entleert, wobei die sich gegebenenfalls im System befindliche Luft entsprechend dem Pfeil 31 entweicht. Der Ballon 1 wird dabei ebenfalls entleert und kollabiert.

Im evakuierten Zustand kann eine Dichtheitsprüfung vorgenommen werden. Insbesondere kann die Dichtheit des Steuer- und Aufblasgeräts, des Ballon und des Katheter sowie ggf. weiterer angeschlossener Volumina überprüft werde, wobei die Komponenten dann als dicht bezeichnet werden können, wenn der evakuierte zustand über eine vorgegebene Zeitdauer gehalten wird.

Befüllung des Systems:
Das System wird nun mit einem Fluid, wie beispielsweise mit Helium, aus dem Fluidvorratsbehälter 30 befüllt. Zu diesem Zweck befinden sich die Ventile 8, 12, 14, 28 und 29 in offenem Zustand. Alle anderen Ventile sind geschlossen. Solange das Absperrventil 29 geöffnet ist, füllt sich das System mit Helium aus dem Vorratstank 30, sodass sich u.a. auch der Druckbehälter 7, die Pumpe 11, die Leitungen 10 und 15 sowie der Vakuumtank 16 gleichmäßig mit Helium füllen.

Vorspannen:
Nach Beendigung des Füllvorgangs wird das Absperrventil 29 geschlossen, und es wird weiters das Ventil 12 geschlossen. Das Ventil 8 bleibt in dem den Druckbehälter 7 mit der Leitung 10 verbindenden Zustand. Um das System vorzuspannen, wird nun die Pumpe 11 in Gang gesetzt, wobei Fluid aus dem Vakuumtank 16 in den Druckbehälter 7 gedrückt wird. Der Vorgang wird solange durchgeführt, bis eine freigegebene Fluidmenge in den Druckbehälter 7 gelangt ist bzw. bis sich eine vorgegebene Druckdifferenz zwischen dem Vakuumtank 16 and dem Druckbehälter 7 einstellt bzw. ein vorgegebener Druck im Druckbehälter 7 erreicht ist. Beispielsweise kann derart vorgegangen werden, dass im Druckbehälter 7 ein Druck von 3 bar erreicht wird, während sich der Druck im Vakuumtank 16 auf 0,6 bar vermindert. Nach Beendigung des Vorspannens des Systems werden alle Ventile geschlossen.

Aufblasen des Ballons:
Zum Aufblasen des Ballons 1 wird nun das Ventil 24 geöffnet,
sodass der Druckbehälter 7 über die Leitung 9 mit dem Anschluss 3 und damit mit dem Ballon 1 verbunden wird, wobei das Ventil 8 geschlossen wird. Zwischen dem Druckbehälter 7 und
dem Ballon 1 findet demzufolge ein Druckausgleich statt, wobei das Ventil 24 solange in der geöffneten Stellung gehalten wird, bis sich ein vollständiger Druckausgleich zwischen dem Druckbehälter 7 und dem Ballon 1 ergibt. Nach dem vollständigen Druckausgleich stehen der Ballon 1 und der Druckbehälter 7 unter demselben Druck, beispielsweise unter einem Druck von 1,2 bar.

Entleeren des Ballons:
Zum Entleeren des Ballons wird das Ventil 22 geöffnet und es wird das Ventil 24 geschlossen, sodass der Druckbehälter 7 vom Anschluss 3 getrennt wird. Der Ballon 1 ist nun direkt mit dem Vakuumtank 16 verbunden, sodass sich aufgrund eines Druckausgleichs zwischen dem Ballon 1 und dem Vakuumtank 16 ein Entleeren des Ballons 1 ergibt. Nach einem vollständigen Druckausgleich stehen der Ballon 1 und der Vakuumtank 16 unter demselben Druck, wobei das Druckniveau beispielsweise 0,8 bar, jedenfalls aber weniger als 1 bar beträgt.

Der Zyklus des Aufblasens und Entleerens des Ballons 1 kann nun beliebig oft wiederholt werden. Zum nochmaligen Aufblasen des Ballons 1 muss das System wieder vorgespannt werden, zu welchem Zweck das Ventil 22 geschlossen wird und das Ventil 14 geöffnet wird. Das Ventil 8 befindet sich wie bereits zuvor beschrieben in geöffnetem Zustand, sodass der Druckbehälter 7 in Verbindung mit der Leitung 10 steht, wobei das Ventil 24 geschlossen ist. Wenn nun die Pumpe 11 in Betrieb genommen wird, wird Fluid aus dem Vakuumtank 16 abgesaugt und in den Druckbehälter 7 gedrückt. Zum Befüllen des Ballons 1 wird wiederum das Ventil 14 geschlossen und die Ventile 8 und 24 derart umgeschaltet, dass der Druckbehälter 7 mit dem Anschluss 3 und dadurch mit dem Ballon 1 verbunden ist. Nach einem Druckausgleich des Druckbehälters 7 und des Ballons 1 ist der Ballon wiederum gefüllt. Die Entleerung des Ballons erfolgt wiederum dadurch, dass die Ventile 8 und 24 umgestellt werden, sowie das Ventil 22 geöffnet wird, wodurch der Ballon 1 mit dem Vakuumbehälter 16 verbunden wird, wodurch Fluid aus dem Ballon 1 in den Vakuumtank 16 fließt, bis ein Druckausgleich zwischen Ballon 1 und Vakuumtank 16 erfolgt ist.

Es ist ersichtlich, dass sowohl das Befüllen des Ballons 1 als auch das Entleeren des Ballons 1 lediglich aufgrund eines vollständigen Druckausgleichs einerseits mit dem Druckbehälter 7 und andererseits mit dem Vakuumtank 16 erfolgt. Das Fluid wird lediglich innerhalb des geschlossenen Systems herumgeführt, sodass sich eine besonders sparsame Betriebsweise ergibt und der Energieverbrauch gesenkt werden kann.

Als Sicherheitsmaßnahme ist vorgesehen, dass die Ventile 8 und 24 hardwaremäßig gegeneinander versperrt sind, sodass sie nicht beide gleichzeitig in geöffnetem oder gleichzeitig in geschlossenem Zustand sein können.

Als weitere Sicherheitsmaßnahme ist vorgesehen, dass das Notfallventil 23 geöffnet wird, wenn auf Grund der von den Druckmesseinrichtungen 4, 5, 13, 32, 33 und 34 gemessenen Druckmesswerte eine Drucküberlast festgestellt wird.

Reinigen des Systems:
Zum Reinigen wird das Ventil 22 geöffnet und das Ventil 8 in eine Stellung gebracht, in welcher der Druckbehälter 7 mit der Leitung 10 verbunden ist. Alle anderen Ventile sind geschlossen. Allfälliges sich im Vakuumtank 16 ansammelndes Kondensat kann in der Folge über die Kondensatpumpe 19 abgepumpt werden.

Das Verfahren zum zyklischen Befüllen des Ballons 1 kann in Form eines Regelalgorithmus realisiert sein, wobei die Realisierung in einer Hardware-Schaltung (elektronische Beschaltung mit Relais und Flipflops) oder Software in einem Mikrocontroller erfolgen kann. Typischer Weise erfolgt die Realisierung in einer Software.

Der Regelalgorithmus kann wie folgt funktionieren:
- Bestimmen des Volumens des Katheters ohne aufgeblasenen Ballon aus mindestens zwei aufeinander folgenden Druckmessungen mit zwei voneinander verschiedenen Druckwerten im Druckbehälter 7. Dazu werden mindestens vier Messwerte (Drücke bei 4 und 5) gemessen.
- Messen des Drucks bei 4 für einen vorgegebenen Druck bei 32.
- Schrittweites Erhöhen des Drucks im Druckbehälter 7 bis ein vorgegebener/gewählter Sollwert für den Druck bei 4 erreicht wird. Dies geschieht in Form eines Regelalgorithmus.

Das Steuer- und Aufblasgerät besteht insgesamt bevorzugt aus einem Mehrprozessorsystem, einem pneumatischen Kreislaufsystem und einem Embedded PC zum Ansteuern eines MMI für die anwenderspezifische Darstellung und Auswertung von Anwendungs- und Messdaten während einer Anwendung. Dabei besteht das Mehrprozessorsystem aus mindestens zwei unabhängigen elektronischen Schaltkreisen, welche sicherheitsrelevante Funktionen ausfallssicher überwachen.

## Patentansprüche

1. Steuer- und Aufblasgerät für einen Ballonkatheter umfassend einen Anschluss (3) für ein Aufblaslumen (2) eines Ballonkatheters, einen Druckbehälter (7), einen Vakuumtank (16) und eine Pumpe (11), wobei der Druckbehälter (7) über eine mit einem Schaltventil (24) versehene Leitung (9) mit dem Anschluss (3) für das Aufblaslumen (2) verbunden ist und der Vakuumtank über eine mit einem Schaltventil (22) versehene Leitung (21) mit dem Anschluss (3) für das Aufblaslumen (2) verbunden ist sodass der Anschluss (3) für das Aufblaslumen (2) abwechselnd mit dem Vakuumtank (16) und mit dem Druckbehälter (7) verbindbar ist, **dadurch gekennzeichnet, dass** die Pumpe (11) in einer den Druckbehälter (7) mit dem Vakuumtank (16) verbindenden Leitung anbeordnet ist, in der ein Sperrventil (14) angeordnet ist, sodass der Druckbehälter (7) abwechselnd mit dem Anschluss (3) für das Aufblaslumen (2) und über die Pumpe (11) mit dem Vakuumtank (16) verbindbar ist.

2. Steuer- und Aufblasgerät nach Anspruch 1, **dadurch gekennzeichnet, dass** ein Anschluss für ein vom Aufblaslumen (2) gesondertes Lumen (6) des Katheters vorgesehen ist, der mit einer Druckmesseinrichtung (5) verbunden ist.

3. Steuer- und Aufblasgerät nach Anspruch 1 oder 2, **dadurch gekennzeichnet, dass** der Druckbehälter (7), die Pumpe (11) und der Vakuumtank (16) mit dem Aufblaslumen (2) des Katheters einen geschlossenen Fluidkreislauf ausbilden.

4. Steuer- und Aufblasgerät nach Anspruch 3, **dadurch gekennzeichnet, dass** der geschlossene Kreislauf einen Anschluss zum Befüllen des Kreislaufes mit Fluid aufweist.

5. Steuer- und Aufblasgerät nach einem der Ansprüche 1 bis 4, **dadurch gekennzeichnet, dass** an den Vakuumtank (16) eine Kondensatpumpe (19) angeschlossen ist.

## Claims

1. A control and inflation device for a balloon catheter, comprising a connection (3) for an inflation lumen (2) of a balloon catheter, a pressure vessel (7), a vacuum tank (16) and a pump (11), wherein by way of a line (9) comprising a control valve (24) the pressure vessel (7) is connected to the connection (3) for the inflation lumen (2), and by way of a line (21) comprising a control valve (22) the vacuum tank is connected to the connection (3) for the inflation lumen (2) so that the connection (3) for the inflation lumen (2) is alternatingly connectable to the vacuum tank (16) and to the pressure vessel (7), **characterised in that** the pump (11) is arranged in a line that connects the pressure vessel (7) to the vacuum tank (16), in which line a shutoff valve (14) is arranged so that the pressure vessel (7) is alternatingly connectable to the connection (3) for the inflation lumen (2) and, by way of the pump (11), to the vacuum tank (16).

2. The control and inflation device according to claim 1, **characterised in that** a connection for a lumen (6) of the catheter, which lumen (6) is separate of the inflation lumen (2), is provided, which connection is connected to a pressure measuring device (5).

3. The control and inflation device according to claim 1 or 2, **characterised in that** the pressure vessel (7), the pump (11) and the vacuum tank (16) form a closed fluid circulation system with the inflation lumen (2) of the catheter.

4. The control and inflation device according to claim 3, **characterised in that** the closed circulation system comprises a connection for filling the circulation system with fluid.

5. The control and inflation device according to any one of claims 1 to 4, **characterised in that** a condensate pump (19) is connected to the vacuum tank (16).

## Revendications

1. Appareil de guidage et de gonflage pour un cathéter à ballonnet, comprenant une connexion (3) pour une voie de gonflage (2) d'un cathéter à ballonnet, un récipient sous pression (7), un récipient sous vide (16) et une pompe (11), sachant que le récipient sous pression (7) est relié à la connexion (3) pour la voie de gonflage (2) via un tuyau (9) doté d'une soupape de commutation (24) et que le récipient sous vide est relié à la connexion (3) pour la voie de gonflage (2) via un tuyau (21) doté d'une soupape de commutation (22), de telle sorte que la connexion (3) pour la voie de gonflage (2) peut être reliée en alternance avec le récipient sous vide (16) et le récipient sous pression (7), **caractérisé en ce que** la pompe (11) est disposée dans une ligne reliant le récipient sous pression (7) au récipient sous vide (16), dans laquelle une soupape d'arrêt (14) est disposée, de telle sorte que le récipient sous pression (7) puisse être relié en alternance avec la connexion (3) pour la voie de gonflage (2) et avec le récipient sous vide (16) via la pompe (11).

2. Appareil de guidage et de gonflage selon la revendication 1, **caractérisé en ce qu'**une connexion pour une voie (6) du cathéter différenciée de la voie de gonflage (2) est prévue, laquelle est reliée à un manomètre (5).

3. Appareil de guidage et de gonflage selon la revendication 1 ou 2, **caractérisé en ce que** le récipient sous pression (7), la pompe (11) et le récipient sous vide (16) forment un circuit de fluide fermé avec la voie de gonflage (2) du cathéter.

4. Appareil de guidage et de gonflage selon la revendication 3, **caractérisé en ce que** le circuit fermé présente une connexion pour remplir le circuit avec du fluide.

5. Appareil de guidage et de gonflage selon l'une des revendication 1 à 4, **caractérisé en ce qu'**une pompe à condensat (19) est raccordée au récipient sous vide (16).
